# EUROPEAN PATENT APPLICATION

(11) **EP 4 191 565 A1**
(43) Date of publication of application: **07.06.2023**
(21) Application number: 21212319.4
(22) Date of filing: 03.12.2021
(51) Int. Cl.: G09B 23/28, A61B 1/00

(54) **ENDOSCOPE IMAGE PROCESSING DEVICE**

(71) Applicant: Ambu A/S, 2750 Ballerup (DK)
(72) Inventor: ALONSO DÍAZ, Alejandro, 2750 Ballerup (DK); GADE, Josefine Dam, 1973 Frederiksberg (DK); JØRGENSEN, Andreas Härstedt, 2610 Rødovre (DK); LASSEN, Lee Herluf Lund, 2760 Måløv (DK); YU, Dana Marie, 2750 Ballerup (DK)
(74) Representative: AWA Denmark A/S

(57) **Abstract**

Disclosed is an image processing device for determining the location of an endoscope in a phantom model. The phantom model is a physical model of a cavity of a human body. The image processing device comprises a processing unit operationally connectable to an image capturing device of an endoscope. The image processing device is configured to obtain a stream of images from the image capturing device of the endoscope. The image processing device is further configured to continuously process images of the stream of images to determine if a predetermined reference position has been reached. The image processing device is further configured to, in response to determining that the predetermined reference position has been reached, updating an estimated location of the endoscope. Further disclosed is a method for determining the location of an endoscope in a phantom model, a display unit connectable to the processing unit, an endoscope system comprising an endoscope and the image processing device, and a computer program product.

## Description

### Field

The present disclosure relates to an image processing device for determining the location of an endoscope in a phantom model, display unit comprising an image processing device, an endoscope system comprising an endoscope and the image processing device, a method for determining the location of an endoscope in a phantom model, and a computer program product.

### Background

Endoscopies of humans are frequently carried out by medical personnel, such as doctors, to examine human cavities. For instance, bronchoscopies or colonoscopies may be carried out to examine whether a patient has a lung or colon disease, respectively, a tumour, or the like, and in some cases samples may be taken/removed from or inserted into part of the airways or colon, respectively. The endoscope typically comprises an image capturing device, such as a camera, at a distal end of the endoscope to be inserted into the patient and connected to a display so as to provide the medical personnel with a view of the part of the cavity, in which the distal end of the endoscope is positioned.

Typically, when an endoscopy is carried out, the medical personnel operate and navigate the endoscope through the human cavity. The endoscope may often need to be navigated through all parts of a human cavity to ensure a sufficient examination of the human cavity. For instance, for bronchoscopies most or all parts of the human lung tree may need to be examined.

When operating and navigating the endoscope, however, the medical personnel often rely on training and experience to navigate the endoscope through the human cavity, e.g. to reach all parts of the human cavity or a specific desired part thereof, based on images from an image capturing device of the endoscope. However, since parts of human cavities, such as human airways or a colon, often look rather similar, it may be difficult for the medical personnel to determine a location of the endoscope. This leads to a risk of mistakes, e.g. in that the desired parts of the human cavity are not examined or in that a part of the airways are mistaken for a different part in the cavity. This, in turn, increases a risk that the patient is not properly examined.

While medical personnel are often trained to perform the endoscope navigation using e.g. phantom models of the human cavities, this training is often based on a "best practice" of an experienced person, such as a doctor, training a less experienced person. This, again, increases the risk of an inconsistent and/or insufficient training, in turn leading to an increased risk of insufficient or incorrect examinations.

In recent years, devices and systems have been provided to aid the medical personnel in navigating the endoscope through the human cavity. While these systems can aid the medical personnel in navigating the endoscope through the human cavity, the systems may themselves require training for the medical personnel to use correctly or optimally.

On this background, it is desirable to provide an image processing device which at least mitigates some of the of the above-mentioned drawbacks.

### Summary

A first aspect of the present disclosure relates to an image processing device for determining the location of an endoscope in a phantom model, the phantom model being a physical model of a cavity of a human body, wherein the image processing device comprises a processing unit operationally connectable to an image capturing device of an endoscope, the processing unit being configured to:
obtain a stream of images from the image capturing device of the endoscope;
continuously process images of the stream of images to determine if a predetermined reference position has been reached;
in response to determining that the predetermined reference position has been reached, updating an estimated location of the endoscope.
It has been realised that by providing an image processing device for determining the location of an endoscope in a phantom model, the location of an endoscope in a phantom model may be obtained by a user. As an example, the user, such as a doctor with limited experience in endoscopy, may be provided with information regarding the location of the endoscope in the phantom model. This, again, may allow for a reliable training of the user as the user may be provided with images from the endoscope, i.e. from an image capturing device of the endoscope, while being provided with the determined estimated location of the endoscope in the phantom model. Thereby, the user may be allowed to associate the images of the endoscope, i.e. the view of the endoscope, with a determined location thereof. This may, again, allow for an improved training of the user, such as the inexperienced doctor, or may allow for improved training of skilled medical personnel, such as a doctor with routine in endoscopies, with respect to using navigation systems during endoscopies.

By the image processing device being configured to process images to determine if a predetermined reference position has been reached, further devices, such as echo signalling devices or the like, to determine a location of the endoscope in the phantom model may be dispensed with. This may, in turn, allow for a simpler system and make handling of the equipment during the navigation of the endoscope in the phantom model simpler, as no additional devices may need to be handled simultaneously with the navigation of the endoscope.

A predetermined reference position may be any position within the phantom model. A predetermined reference position may be and/or may correspond to a position, which the endoscope such as a distal end thereof, can take in the phantom model. In some embodiments, the predetermined reference position is a position at which a visual characteristic occurs, which allows the image processing device to estimate, based on the image, the position of the endoscope. Alternatively or additionally, a predetermined reference position may be a position, at which a furcation occurs in the phantom model. For instance where the phantom model represents the human airways, a furcation may occur at the position in the phantom model representing the position in the human airways, at which the trachea bifurcates into the left and right bronchus, and/or where bronchi and/or bronchioles furcate.

The estimated endoscope location may correspond to or be a part or segment of the phantom model, in which the endoscope is determined to be.

The endoscope location may be a location of a tip part of an endoscope. Alternatively or additionally, the endoscope location may be a location of the endoscope in a phantom model, such as a cavity of the phantom model, and/or in a model, e.g. a computer model, of the phantom model. The endoscope location may be or indicate a segment of the phantom model.

In some embodiments, the processing unit may be configured add to the image(s) an estimated endoscope location, potentially as an overlay.

The phantom model may be a physical model of the human lung tree or of the human colon. The phantom model may resemble and/or model the human cavity

The phantom model may comprise one or more visual markers. The one or more visual markers may be arranged in the phantom model, potentially so that the one or more visual markers can be recognised in images from the endoscope. The one or more visual markers, such as each visual marker may comprise a QR code, a texture pattern, a pattern of varying colour and/or contrast. The one or more visual markers may be configured to be recognisable by the image capturing device and/or by the image processing device from the images obtained by the image capturing device. The one or more visual markers may be suitable for and/or configured to allow the image processing device to provide an augmented image based on the visual markers.

In some embodiments, the processing unit may be any processing device/unit, such as a central processing unit (CPU), a graphics processing unit (GPU), a microcontroller unit (MCU), a field-programmable gate array (FPGA), or any combination thereof.

The image processing unit may comprise a display, i.e. the image processing device may be integrated in a display unit. Alternatively or additionally, the image recording unit may be operationally connectable to a separate display unit.

In some embodiments, the processing unit may further be configured to store one or more earlier estimated locations of the endoscope. Alternatively or additionally, the processing unit may be configured to estimate a location, such as a current location, of the endoscope in the phantom model based on the image(s) from the image stream and at least one earlier estimated location.

Alternatively or additionally, the processing unit may be configured to provide guidance to the user, potentially so as to guide to user to navigate the endoscope to a predetermined location in the phantom model and/or guide the user to navigate the endoscope to a plurality of predetermined location in a specific predetermined order. Thereby, the user may be assisted in carrying the endoscopic procedure, which the user performs in the phantom model, according to an established "best practice". The processing unit may be configured to provide the guidance based on a determined estimated location of the endoscope and/or, where one or more earlier estimated locations of the endoscope is stored, based on at least one of the one or more earlier estimated location.

Where an output signal is provided by the processing unit, the output signal may comprise the guidance, potentially in the form of instructions and/or one or more predetermined location(s).

In some embodiments, where the phantom model comprises one or more visual markers, the image processing device may be configured to analyse an image from the stream of images to determine if one or more visual markers are present in the image. The image processing device may further be configured to, in response to determining that one or more visual markers are present in the image, determine the location of the endoscope based on the determined one or more visual markers. Alternatively or additionally, the image processing device may further be configured to, in response to determining that one or more visual markers are present in the image, augment the image based on the determined one or more visual markers.

A further aspect of the present disclosure relates to a phantom model, the phantom model being a physical model of a cavity of a human body, wherein the phantom model comprises one or more visual markers. The one or more visual markers may be suitable for being obtained by an image capturing device of an endoscope, when the endoscope is navigated in the phantom model, such as in an interior of the phantom model.

In some embodiments, the image processing device is configured to continuously process the images using a machine learning data architecture to determine if a reference position has been reached, the machine learning data architecture being trained to determine a predetermined reference position.

Thereby, a robust recognition of the predetermined reference positions may be provided.

The machine learning data architecture may be a first machine learning data architecture.

The (first) machine learning data architecture may be trained by being provided with a training data set comprising a number, such as a large number, such as 100 or more, image streams, each potentially comprising a plurality of images, from an endoscope and having a human operator determine the predetermined reference position, at which the images have been obtained in the phantom model. The training data set may comprise one or more images showing reference positions inside a phantom model, such as inside a cavity of the phantom model. The images may be from a video stream of an image device of an endoscope. The machine learning data architecture may be trained to optimise towards a F score, such as a F1 score or a Fβ, which it will be appreciated is well known in the art. The machine learning data architecture may be trained using the training data set and corresponding associated predetermined reference positions. Potentially, the anatomic reference positions may be associated by a plurality of people.

While phantom may models resemble and/or model a human cavity, the textures, shapes, and/or features from the phantom models are typically somewhat different from that of a real human cavity. Thus, by training the machine learning data architecture using images obtained in a phantom model, a more robust and/or reliable determination of an endoscope location in a phantom model may be provided by the machine learning data architecture.

In some embodiments, the reference position may be a reference position of a subset of predetermined reference positions. The subset of predetermined reference position may be a subset from a set of predetermined reference positions. The set may comprise the subset of predetermined reference positions and at least one further reference position.

In some embodiments, a subset of predetermined reference positions comprises two or more reference positions from the set of predetermined reference positions, such as some but not all of the predetermined reference positions from the set of predetermined reference positions.

In some embodiments, the image processing device may be configured to determine a subset of predetermined reference positions based on the estimated endoscope location, such as a previously estimated endoscope location.

In some embodiments, the machine learning data architecture may alternatively or additionally be trained to determine a set of predetermined reference positions. A set of predetermined reference positions may comprise two or more predetermined reference positions.

In some embodiments, the image processing device may be configured to detect openings and/or lumens in an image of the image stream.

In some embodiments, the determination whether a predetermined position has been reached further comprises to continuously analyse the recorded images to determine if two or more lumens, potentially of a branching structure, are present in at least one of the recorded images. The determination whether two or more lumens are present may be carried out by the (first) machine learning data architecture. Alternatively, the determination if two or more lumens are present in the at least one recorded image may be performed using a second machine learning architecture trained to detect lumens in an endoscope image.

In some embodiments, for instance where the phantom model is a model of a human lung tree, the processing unit may be configured to estimate a location of the endoscope in response to a determination that two or more lumens are present in the at least one recorded image. Alternatively or additionally, a predetermined reference position may be a position, at which a branching occurs, i.e. where two or more lumens are present in the at least one recorded image.

The processing unit may be configured to, where it is determined that two or more lumen are present in the image(s), determine a location of these in the phantom model. The processing unit may further be configured to indicate a location of the two or more lumen, such as which part of the phantom model, the endoscope will enter if entering either of the two or more lumen. For instance, where the phantom model is a model of the human lung tree and two lumens are detected, the image processing unit may determine that the two lumen lead to a phantom model portion modelling a left and phantom model portion modelling a right main bronchus, respectively, and indicate this, e.g. by an overlay on each of the two lumen.

In some embodiments, the image processing device may further be configured to, in response to determining that two or more lumens are present in the at least one recorded image: determining which one of the two or more lumens the endoscope enters; and updating an estimated endoscope location based on the determined one of the two or more lumens. In some embodiments, determining which one of the two or more lumens the endoscope enters may be performed by analysing, in response to a determination that two or more lumens are present in the at least one recorded image, a plurality of recorded images to determine a movement of the endoscope.

The processing unit may, where this is configured to use a machine learning data architecture, such as a first, a second, and/or a third machine learning data architecture, implement the machine learning data architecture, i.e. the processing unit may provide the image to the machine learning data architecture by processing the image using the machine learning data architecture. Alternatively, the processing unit may provide the image, a parameterized version of the image, or a dimensionally reduced version of the image to another processing unit e.g. outside of the image processing device, where said another processing unit implements the machine learning data architecture.

In some embodiments the phantom model is a three-dimensional model of the human body cavity.

The three-dimensional (3D) model of human cavity may be a 3D model of the human airways or colon. The 3D model may be configured to resemble the human cavity. The 3D model may be configured to receive an endoscope.

The three-dimensional model of the human body cavity may be made from a polymer material. The three-dimensional model may be approximately scale 1:1 of the human cavity, which it models.

In some embodiments the image processing device is further configured to:
output an output signal based at least in part on one or more of the images of the stream of images.

Thereby, the estimated endoscope location, the images from the endoscope, and/or other data based on the images may be signalled to another device.

The output signal may comprise one or more images of the stream of images. Alternatively or additionally, the output signal may comprise the estimated endoscope location and/or may comprise an indication, such as a coordinate, a text, and/or a visual marker or overlay, indicating the estimated endoscope location.

The image processing device may comprise an output signal port for outputting the output signal. The output signal port may comprise a plug and/or a socket for interconnection with another device and/or may comprise a wireless communication interface.

The output signal may be configured to be received by a display unit and/or may be configured to be displayed on a display unit.

In some embodiments the image processing device is further configured to:
augment an image from the stream of images by obtaining a computer model representing at least a part of the phantom model, determining an estimated position of the endoscope relative to the part of the phantom model, and based on the estimated position and the computer model, augmenting the image, wherein the output signal is based at least in part on the augmented image.

Thereby, further information, such pathological conditions, may be emulated, thereby providing an improved training of the user, since the user may react to these.

The computer model may be determined based on one or more image(s) from the stream or images and/or a structure of the phantom model. The computer model may represent a geometry of the part of the phantom model, may be and/or comprise a plane. Additionally or alternatively, the computer model may represent a three-dimensional geometry of the part of the phantom model.

In some embodiments, the computer model may comprise a simulation of a pathological condition.

The computer model may be selected between a plurality of computer models and/or may be, at least pseudo-, randomly generated.

The determined estimated position relative to the part of the phantom model may have same accuracy as the estimated endoscope location in the phantom and/or may be more accurate. The estimated position relative to the part of the phantom model may be determined based on image(s) and/or based on visual markers, where visual markers are provided in the phantom model. Additionally or alternatively, the estimated position may be based on knowledge and/or data about the phantom model. The knowledge and/or data may be structural knowledge of the phantom model, such as information from a CT scan thereof, information regarding the three-dimensional geometry, and/or manufacturing data, such as CAD data, of the phantom model.

The output signal may alternatively or additionally be and/or may comprise the augmented image, potentially configured to be displayed on a display unit.

The augmentation of the image may comprise adding features, potentially in an overlay, to the image, such as a polyp, a scratch, a section with specific colour, and/or a section with specific surface.

In some embodiments the computer model comprises a virtual light source simulating light from one or more light sources of the endoscope.

Thereby a more realistic augmentation of the image may be provided.

The virtual light source may be based on an orientation and/or a position of the endoscope. Light from the virtual light source may be determined, potentially by the image processing device, based on the images.

In some embodiments the augmenting the image comprises adding to the image an overlay resembling human tissue.

The overlay may be based on visual markers, where the phantom model comprises visual markers.

In some embodiments, the image processing device may be configured to augment the image to include a virtual surface, such as a three-dimensional surface. The virtual surface may be adapted to resemble or imitate a polyp.

In some embodiments the image processing device is configured to augment the image based on information about the computer model, the information being based on physical properties of the phantom model.

Thus, the geometry of the phantom model near the section, at which the image was obtained may be taken into account when an augmentation is to, thereby allowing for a more realistic augmentation of the image.

The information about the computer model may be information relating to the geometry of the phantom model. For instance, the information may be based on manufacturing information, such as CAD information or 3D print information, a CT scan of the phantom model, or a MR scan of the phantom model.

In some embodiments, the computer model may be a rendering of the phantom model and/or may be synthesised based on the phantom model. The computer model may be a virtual representation of the phantom model or an estimated virtual representation of the phantom model. In some embodiments, the augmentation of the image may be based on a synthesised image from the computer model. For instance, the image processing device may, based on the computer model, be configured to generate a synthesised image, which estimates an image which would be obtained from the endoscope, when the endoscope is at a given location in the model. Alternatively or additionally, the image processing device may be configured to generate a synthesised outcome space, potentially comprising a plurality of synthesised images and, based on a match between the image of the image stream and the synthesised images, determine an estimated location of the endoscope and/or augment the image.

In some embodiments the image processing device is further configured to determine, based on an image of the image stream, if a tool is visible in the image and, if a tool is visible, augment the image based on the determination that a tool is visible.

Thereby, the augmented images may allow for simulating a biopsy from the phantom model, again allowing for an improved training of the user.

In some embodiments, the image processing device may be configured to determine the type of tool and augment the image based on the type of tool. Alternatively or additionally, the image processing device may determine a position of the tool in the image and augment the image accordingly.

The image processing device may be configured to determine if a tool is visible in the image using a, potentially third, machine learning data architecture trained to determine if a tool is visible. The machine learning data architecture may be trained by providing to the machine learning data architecture a training data set comprising a plurality, such as a large number, of images obtained by an endoscope, and by a user classifying if a tool is visible in the image or not. In some embodiments, the image processing device may be configured to identify a tool, i.e. a type of tool, potentially using the (third) machine learning data architecture and/or a visual marker provided on the tool. In some embodiments, the image processing device may further be configured to augment the image in response to an identification of the tool.

In some embodiments the image processing device is further configured to:
estimate a quality parameter of a navigation of the endoscope in the phantom model based on the images of the image stream.

Thereby, a quality of the endoscopy may be provided to the user, again allowing for an improved training of the user.

The quality parameter may be based on, comprise, and/or be an estimate of the time the endoscope has spent in a segment or at an estimated location, and/or an estimate of if all locations from a set of locations in the phantom model have been visited. Alternatively or additionally, the quality parameter may be based on a determination whether a navigation of the endoscope in the phantom model would have caused harm to a patient, if the endoscope had been navigated identically in a human cavity, which the phantom model resembles.

A second aspect of the present disclosure relates to a display unit for displaying images obtained by an image processing device of an endoscope, wherein the display unit comprises an image processing device according to the first aspect.

A third aspect of the present disclosure relates to an endoscope system comprising an endoscope and an image processing device according to the first aspect, wherein the endoscope has an image capturing device and the processing unit of the image processing device is operationally connectable to the image capturing device of the endoscope.

In some embodiments, the endoscope system further comprises a display unit, wherein the display unit is operationally connectable to the image processing device.

In some embodiments, in which the image processing device outputs an output signal, the display unit may be configured to receive the output signal from, potentially as an input signal to the display unit.

In some embodiments of the endoscope system the image processing device forms part of a display unit according to the second aspect.

A fourth aspect of the present disclosure relates to a method for determining the location of an endoscope in a phantom model, the phantom model being a physical model of a cavity of a human body, wherein the method comprises:
obtaining a stream of images from an image capturing device of an endoscope;
continuously processing images of the stream of images to determine if a predetermined reference position has been reached;
in response to determining that a predetermined reference position has been reached, updating an estimated location of the endoscope.

A fifth aspect of the present disclosure relates to a computer program product comprising program code means adapted to cause a data processing system to perform the steps of the method according to the fourth aspect of this disclosure, when said program code means are executed on the data processing system.

In some embodiments, the computer program product comprises a non-transitory computer-readable medium having stored thereon the program code means.

The different aspects of the present disclosure can be implemented in different ways including image processing devices, display units, endoscope systems, methods for determining the location of an endoscope in a phantom model, and computer program products described above and in the following, each yielding one or more of the benefits and advantages described in connection with at least one of the aspects described above, and each having one or more preferred embodiments corresponding to the preferred embodiments described in connection with at least one of the aspects described above and/or disclosed in the dependent claims. Furthermore, it will be appreciated that embodiments described in connection with one of the aspects described herein may equally be applied to the other aspects.

### Brief description of the drawings

The image processing device will now be described in greater detail based on non-limiting exemplary embodiments and with reference to the drawings, of which:
FIG. 1a shows a perspective view of an endoscope in which a tip part assembly according to the present disclosure is implemented,
FIG. 1b shows a perspective view of a display unit to which the endoscope of FIG. 1a is connected,
FIG. 2 shows a flow chart of a method for determining the location of an endoscope in a phantom model according to an embodiment of the disclosure,
FIG. 3 shows a schematic drawing of an image processing device according to an embodiment of the disclosure,
FIG. 4 shows a schematic drawing of an endoscope system according to an embodiment of the disclosure,
FIG. 5 shows a view of a schematic model of a display unit according to an embodiment of the disclosure, and
FIG. 6 shows a view of an image of a display unit according to an embodiment of the disclosure.

### Detailed description

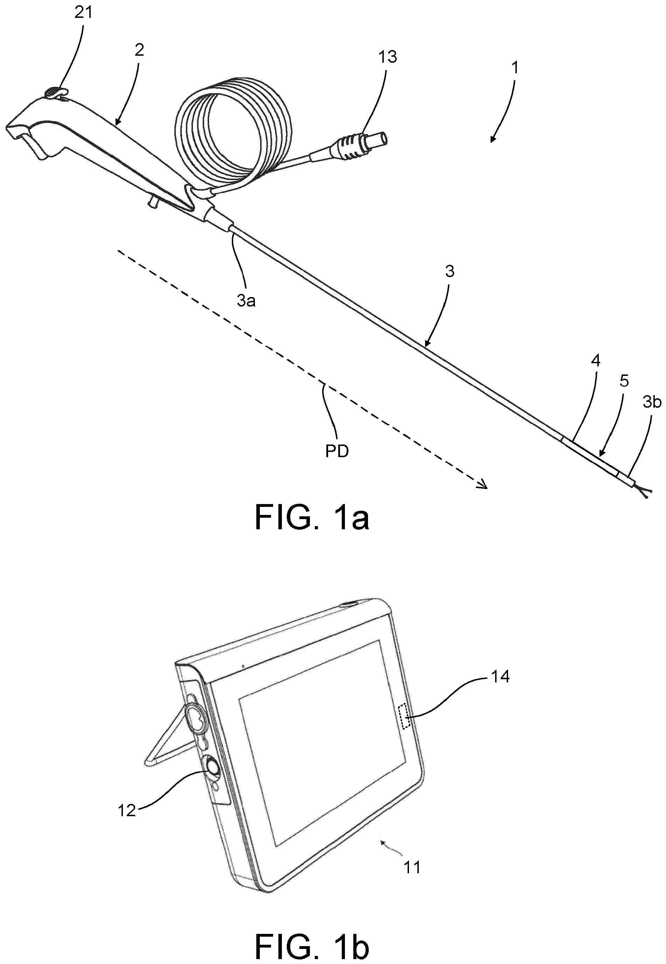

Referring first to FIG. 1a, an endoscope 1 is shown. In all embodiments, the endoscope may be disposable and may not be intended to be cleaned and reused. Alternatively the endoscope may, in all embodiments, be re-usable. The endoscope 1 comprises an elongated insertion tube 3. At the proximal end 3a of the insertion tube 3 an operating handle 2 is arranged. The operating handle 2 has a control lever 21 for manoeuvring a tip part assembly 5 at the distal end 3b of the insertion tube 3 by means of a steering wire. A camera assembly 6 is positioned in the tip part 5 and is configured to transmit an image signal through a monitor cable 13 of the endoscope 1 to a monitor 11.

In FIG. 1b, a display unit comprising a monitor 11 is shown. The monitor 11 may allow an operator to view an image captured by the camera assembly 6 of the endoscope 1. The monitor 11 comprises a cable socket 12 to which a monitor cable 13 of the endoscope 1 can be connected to establish a signal communication between the camera assembly 6 of the endoscope 1 and the monitor 11. The display unit further comprises an image processing device 14 (only schematically shown).

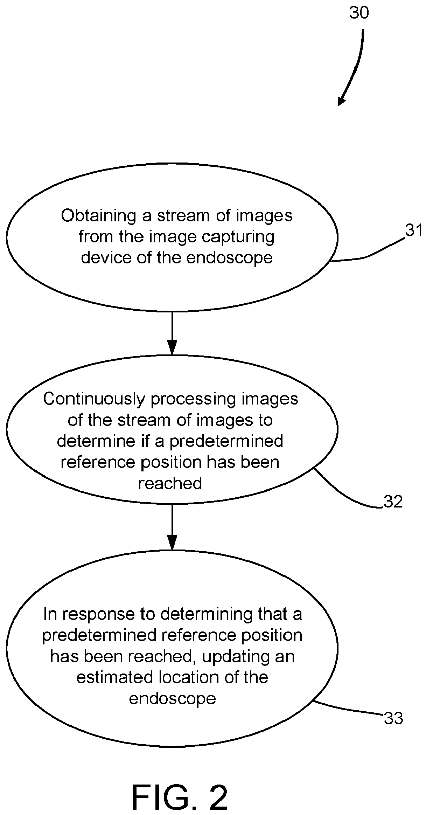

FIG. 2 shows a flow chart of a method 30 for determining the location of an endoscope in a phantom model according to an embodiment of the disclosure. The phantom model is a physical model of a cavity of a human body.

The method 30 initiates at step 31 of obtaining a stream of images from an image capturing device of an endoscope. Next, in step 32, images of the stream of images are continuously processed to determine if a predetermined reference position has been reached. Then, in step 33, an estimated location of the endoscope is updated in response to determining that a predetermined reference position has been reached.

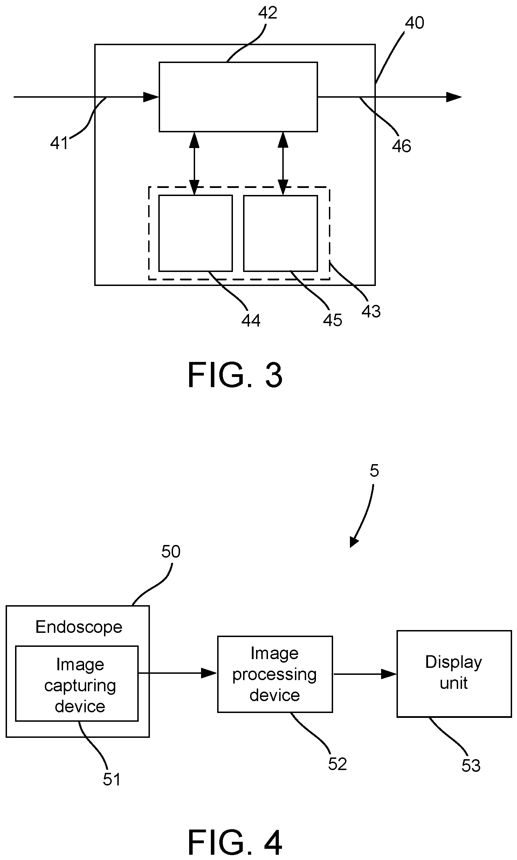

FIG. 3 shows a schematic drawing of an image processing device 40 for recognising previously observed locations during an endoscopic procedure according to an embodiment of the disclosure.

The image processing device 40 comprises a processing unit 42, which is operationally connectable 41 to an image capturing device of an endoscope. The processing unit 42 is configured to obtain a stream of images from the image capturing device of the endoscope during the endoscopic procedure. The processing unit 42 may be wired connectable to the image capturing device. As an example, the image processing device may comprise a connection port for operationally connecting the image capturing device of the endoscope to the processing unit 42. The connection port may further be used to provide power to the endoscope. Alternatively, the processing unit 42 may be wirelessly connectable to the image capturing device e.g. the image processing device 40 may comprise a wireless communication unit (not shown) configured to receive images from the image capturing device. The processing unit 42 is further configured to continuously process images of the stream of images to determine if a predetermined reference position has been reached. The processing unit is further configured to, in response to determining that the predetermined reference position has been reached, updating an estimated location 44 of the endoscope. The updated estimated location 44 is stored in a memory 43 of the image processing device 40. In other embodiments, the updated estimated location 44 may be stored in an external memory and/or an internal memory of a memory device (not shown), to which the processing unit may be connectable, e.g. by a wired and/or a wireless connection. Alternatively or additionally, the updated estimated location 44 may be stored on a server accessible to the processing unit 42 by means of a wired and/or wireless connection.

The processing unit 42 is configured to continuously process the images using a machine learning data architecture 45 to determine if a reference position has been reached, the machine learning data architecture 45 being trained to determine a predetermined reference position. As an example, the machine learning data architecture 45 is stored in the memory 43. In other embodiments, the machine learning data architecture may be stored in an external memory and/or an internal memory of a memory device (not shown), to which the processing unit may be connectable, e.g. by a wired and/or a wireless connection.

The processing unit 42 is furthermore configured to output 46 an output signal indicating and/or comprising information regarding an estimated location of the endoscope. The output signal may be provided to a display unit (not shown).

FIG. 4 shows a schematic drawing of an endoscope system 5 according to an embodiment of the disclosure. The endoscope system 5 comprises an endoscope 50, an image processing device 52 having a processing unit. The endoscope 50 has an image capturing device 51 and the processing unit of the image processing device 52 is operationally connectable to the image capturing device of the endoscope 51. In this embodiment, the image processing device 52 is operationally connectable to a display unit 53. In other embodiments, the image processing device 52 is integrated in the display unit 53. The image processing device 52 is configured to determine a location of an endoscope in a phantom model, the phantom model being a physical model of a cavity of a human body. The image processing 52 device comprises a processing unit (not shown) operationally connectable to the image capturing device 51 of the endoscope 50. The processing unit is configured to obtain a stream of images from the image capturing device 51 of the endoscope 50. The processing unit is further configured to continuously process images of the stream of images to determine if a predetermined reference position has been reached and in response to determining that the predetermined reference position has been reached, updating an estimated location of the endoscope.

The image processing device 52 may be similar and/or identical to the image processing device 40 described with respect to FIG. 4.

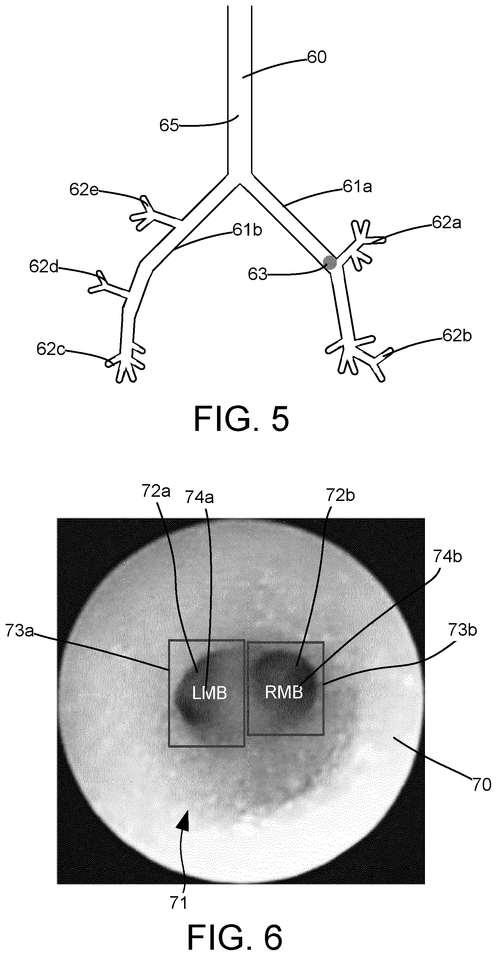

In the view of FIG. 5, a view of a schematic model of a phantom model, the phantom model being a three-dimensional model of human airways, is shown. The schematic model is a computer model of the phantom model. The view shown in FIG. 5 is not necessarily in scale and the relative size of individual parts or elements therein does not necessarily correspond to the relative sizes of the parts or elements of the phantom model which they model. In FIG. 5, the view of the schematic model illustrates a part 60 of the phantom model, which models a trachea, a part 61a, which models the left primary bronchus, and a part 61b, which models a right primary bronchus. The schematic model moreover illustrates parts 62a-62e of the phantom model, which parts 62a-62e model secondary bronchi as well as some bronchioles.

In the view of the schematic model of FIG. 5, an estimated location 63 of the endoscope in the phantom model is shown. The estimated endoscope location 63 is indicated by a dot arranged at the position in the model substantially corresponding to and representing the estimated location of the endoscope in the phantom model. It should be noted that the dot need not show an exact real-time location of the endoscope but may show an approximated location or an area or part of the phantom model, in which the endoscope is estimated to be located. In other embodiments, the estimated endoscope location may alternatively or additionally be indicated by a different graphical symbol than the dot, by text, or the like. The estimated location 63 is determined by the image processing device 40 shown in FIG. 3.

FIG. 6 shows a view of an image 70 of a display unit according to an embodiment of the disclosure. The image 70 of the display unit is based on an image obtained by an image capturing device of an endoscope. Based on the image from the image capturing device of the endoscope, the image processing device identifies a first lumen 72a and a second lumen 72b of a branching 71. The image processing device further determines a bounding box 73a of the first lumen 72a and a bounding box 73b of the second lumen 72b. In other embodiments, the bounding boxes 73a, 73b may be dispensed with and/or not indicated in the image 70.

The image processing device moreover estimates a position of the first lumen 72a as a part of the phantom model, which part models a left main bronchus (LMB), and a position of the second lumen 72b as a part of the phantom model, which part models the right main bronchus (RMB). The image processing device determines this based on the image and/or an estimated location of the endoscope. The image processing device indicates this with a text overlay 74a indicating the estimated position of the first lumen 72a and a text overlay 74b indicating the estimated position of the second lumen 72b. The image processing device may further determine that the endoscope enters a lumen, such as the first lumen 72a, and update the endoscope location to correspondingly, e.g. to LMB. In other embodiments, the text overlays 74a, 74b may be dispensed with and/or not indicated in the image 70.

Although some embodiments have been described and shown in detail, the invention is not restricted to them, but may also be embodied in other ways within the scope of the subject matter defined in the following claims. In particular, it is to be understood that other embodiments may be utilised, and structural and functional modifications may be made without departing from the scope of the present invention.

In device claims enumerating several means, several of these means can be embodied by one and the same item of hardware. The mere fact that certain measures are recited in mutually different dependent claims or described in different embodiments does not indicate that a combination of these measures cannot be used to advantage.

It should be emphasized that the term "comprises/comprising" when used in this specification is taken to specify the presence of stated features, integers, steps or components but does not preclude the presence or addition of one or more other features, integers, steps, components or groups thereof.

## Claims

1. An image processing device for determining the location of an endoscope in a phantom model, the phantom model being a physical model of a cavity of a human body, wherein the image processing device comprises a processing unit operationally connectable to an image capturing device of an endoscope, the processing unit being configured to:
obtain a stream of images from the image capturing device of the endoscope;
continuously process images of the stream of images to determine if a predetermined reference position has been reached;
in response to determining that the predetermined reference position has been reached, updating an estimated location of the endoscope.

2. The image processing device according to claim 1, wherein the image processing device is configured to continuously process the images using a machine learning data architecture to determine if a reference position has been reached, the machine learning data architecture being trained to determine a predetermined reference position.

3. The image processing device according to any one of the preceding claims, wherein the phantom model is a three-dimensional model of the human body cavity.

4. The image processing device according to any one of the preceding claims, wherein the image processing device is further configured to:
output an output signal based at least in part on one or more of the images of the stream of images.

5. The image processing device according to claim 4, wherein the image processing device is further configured to:
augment an image from the stream of images by obtaining a computer model representing at least a part of the phantom model, determining an estimated position of the endoscope relative to the part of the phantom model, and based on the estimated position and the computer model, augmenting the image, wherein the output signal is based at least in part on the augmented image.

6. The image processing device according to claim 5, wherein the computer model comprises a virtual light source simulating light from one or more light sources of the endoscope.

7. The image processing device according to claim 5 or 6, wherein the augmenting the image comprises adding to the image an overlay resembling human tissue.

8. The image processing device according to any one of claims 5-7, wherein the image processing device is configured to augment the image based on information about the computer model, the information being based on physical properties of the phantom model.

9. The image processing device according to any one of claims 5-8, wherein the image processing device is further configured to determine, based on an image of the image stream, if a tool is visible in the image and, if a tool is visible, augment the image based on the determination that a tool is visible.

10. The image processing device according to any one of the preceding claims, wherein the image processing device is further configured to:
estimate a quality parameter of a navigation of the endoscope in the phantom model based on the images of the image stream.

11. A display unit for displaying images obtained by an image processing device of an endoscope, wherein the display unit comprises an image processing device according to any one of claims 1-10.

12. An endoscope system comprising an endoscope and an image processing device according to any one of claims 1-10, wherein the endoscope has an image capturing device and the processing unit of the image processing device is operationally connectable to the image capturing device of the endoscope.

13. The endoscope system according to claim 12 further comprising a display unit, wherein the display unit is operationally connectable to the image processing device.

14. An endoscope system according to claim 13, wherein the image processing device forms part of a display unit according to claim 11.

15. A method for determining the location of an endoscope in a phantom model, the phantom model being a physical model of a cavity of a human body, wherein the method comprises:
obtaining a stream of images from an image capturing device of an endoscope;
continuously processing images of the stream of images to determine if a predetermined reference position has been reached;
in response to determining that a predetermined reference position has been reached, updating an estimated location of the endoscope.

16. A computer program product comprising program code means adapted to cause a data processing system to perform the steps of the method according to claim 15, when said program code means are executed on the data processing system.

17. The computer program product according to claim 16, wherein said computer program product comprises a non-transitory computer-readable medium having stored thereon the program code means.
